# EUROPEAN PATENT APPLICATION

(11) **EP 1 647 221 A1**
(43) Date of publication of application: **19.04.2006**
(21) Application number: 04748016.5
(22) Date of filing: 22.07.2004
(51) Int. Cl.: A61B 1/00

(54) **ENDOSCOPE**

(30) Priority: 22.07.2003 JP 2003200155
(71) Applicant: Olympus Corporation, Tokyo 151-0072 (JP)
(72) Inventor: MORIYAMA, Hiroki, Shibuya-ku, Tokyo 151-0072 (JP)
(74) Representative: von Hellfeld, Axel
(86) International application number: PCT/JP2004/010751
(87) International publication number: WO 2005/006965

(57) **Abstract**

An endoscope 1 of the present invention comprises an inserting portion having a bending portion which can bend in a plurality of directions and an operation section having multiple bending operation members 23 for operating the bending portion of the inserting portion. In the endoscope 1, at least one bending direction of the bending portion operated by a bending operation member 23A in the vicinity of a handle 3a of the operation section among the multiple bending operation members 23 is provided such that the length of the bending part thereof is longer than that in other bending direction operated by another bending operation member 23B. Further, preferably, the bending direction of the bending portion operated by the bending operation member 23A closer to the handle 3a is the one to move an observation image that is displayed on a monitor as display means in the gravity direction or anti-gravity direction of the monitor.

## Description

### Technical Field

The present invention relates to an endoscope including an inserting portion having a bending portion bendable in multiple directions.

### Background Art

Hitherto, endoscopes have been widely used. An endoscope may be used to observe an organ in a body cavity and the like by inserting an elongated inserting portion into the body cavity. An endoscope may also be used to perform various treatments by using a therapeutic instrument inserted into a therapeutic-instrument-through channel as required.

Among these conventional endoscopes, Japanese Examined Patent Application Publication No. 51-35794 discloses an endoscope including an inserting portion having a bending portion bendable in multiple directions such as vertical and horizontal directions.

The endoscope disclosed in the publication includes a bending portion to be bent into different lengths in accordance with bending directions such that the bending portion can bend greatly in one direction and small in another direction. Thus, a bend radius can be selected in accordance with a given condition.

Generally, in an endoscope, a bending operation wire is pulled and loosened in response to a manipulation on a bending operation member such as a knob and a lever in an operation section. Thus, bending operations of the bending portion can be performed.

In accordance with the Japanese Examined Patent Application Publication No. 51-35794, however, there is not described the relation of the operability by the bending operation member and the different bending radiuses of the bending portion, i.e. as to in which directions in concrete the bending portion should bend large and small.

The present invention has been made under the circumstances and is to provide an endoscope which can keep good the operability at the time of inserting and select among bending directions for different bending radiuses as needed.

### Disclosure of Invention

An endoscope according to the invention includes an inserting portion to be inserted into a subject, a bending portion provided in the inserting portion for being bent in accordance with a bending operation by an operator, a first bending operation member provided in an operation section for bending the bending portion in at least one direction, and a second bending operation member provided in the operation section for bending the bending portion in a different direction from the at least one direction. In this case, the first bending operation member is provided closer to a part grasped by the operator than the second bending operation member with respect to the operation section, and a bending range of the bending portion bent in accordance with a manipulation on the first bending operation member is larger than a bending range of the bending portion bent in accordance with a manipulation on the second bending operation member.

### Brief Description of the Drawings

Fig. 1 is a construction diagram showing an endoscope according to an embodiment of the invention;
Fig. 2 is a front view of a distal-end portion in Fig. 1;
Fig. 3 is a schematic perspective view showing a construction of a bending portion in Fig. 1;
Fig. 4 is a view of the bending portion in the direction indicated by the arrow B in Fig. 3;
Fig. 5 is a view of the bending portion in the direction indicated by the arrow C in Fig. 3;
Fig. 6 is a view of the endoscope in the direction indicated by the arrow A in Fig. 1;
Fig. 7 is a schematic explanatory diagram in which a direction of an observation image displayed on a monitor is illustrated;
Fig. 8 is a schematic explanatory diagram showing a state of an inserting portion having a bending portion bending at a small bend radius in a winding portion of a lumen;
Fig. 9 is a schematic explanatory diagram showing a state of the inserting portion having the bending portion bending at a large bend radius in the winding portion of the lumen;
Fig. 10 is a schematic explanatory diagram showing a state of the inserting portion having the distal-end portion near a target part within a lumen; and
Fig. 11 is a schematic explanatory diagram showing a state of the inserting portion having the bending portion bending from the state in Fig. 10 at a small bend radius.

### Best Mode for Carrying Out the Invention

An embodiment of the present invention will be described below with reference to drawings.

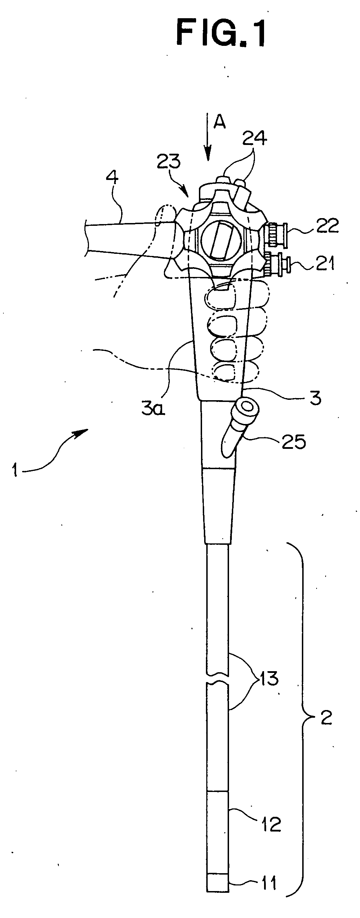

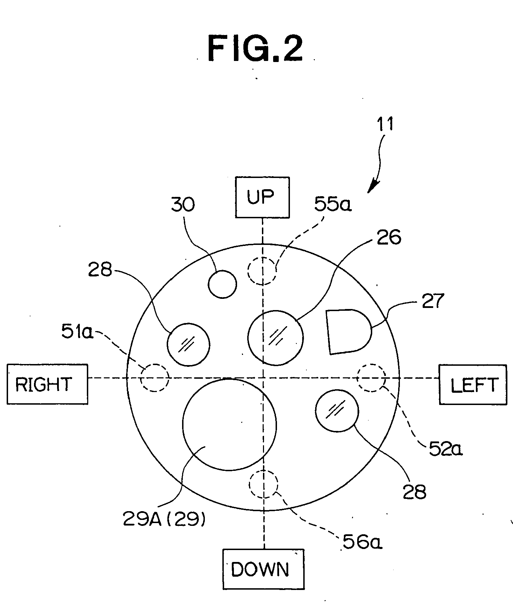

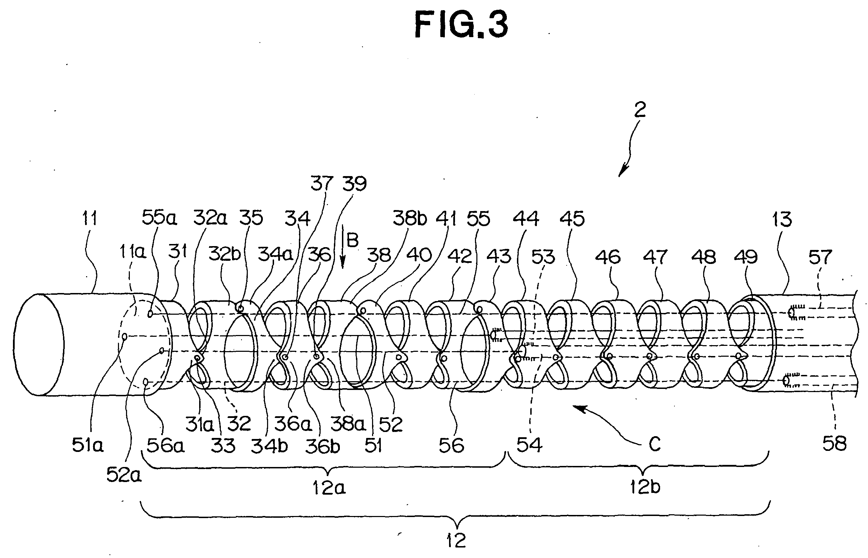

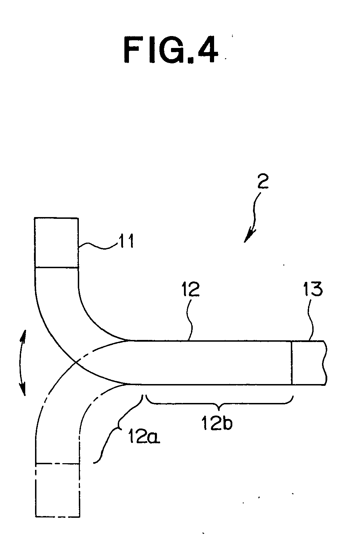

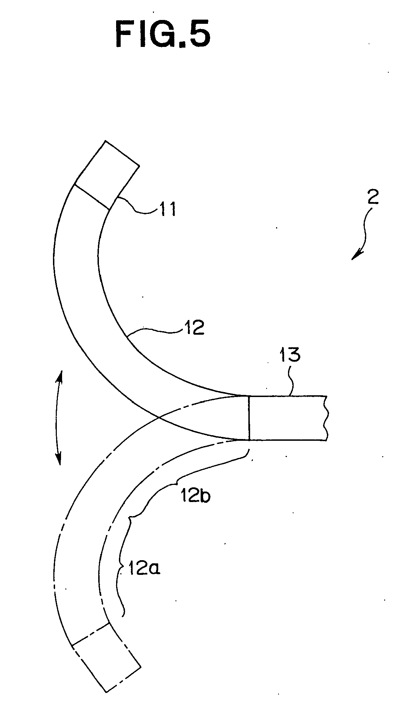

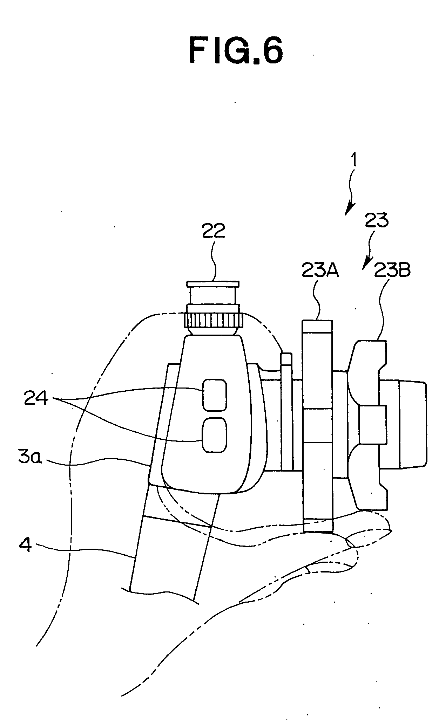

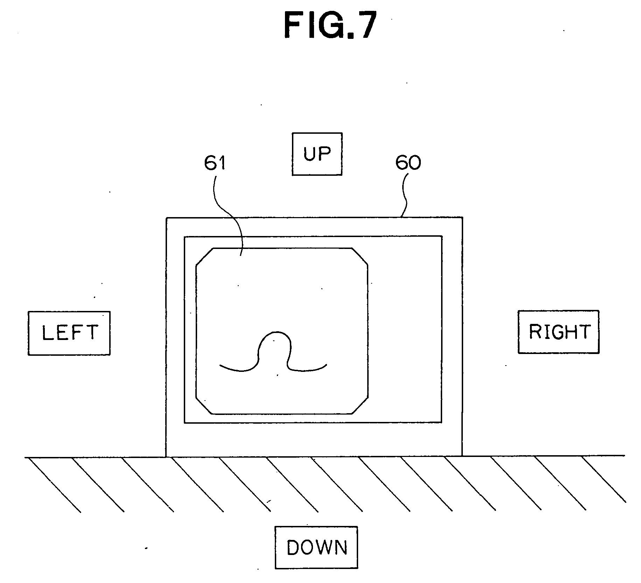

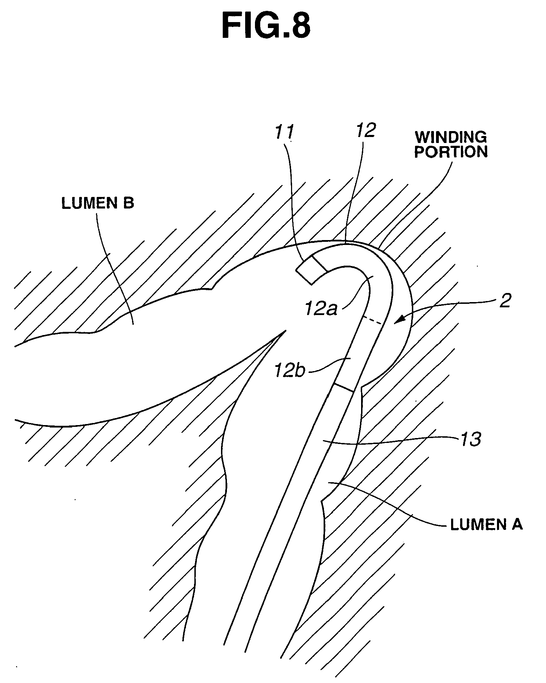

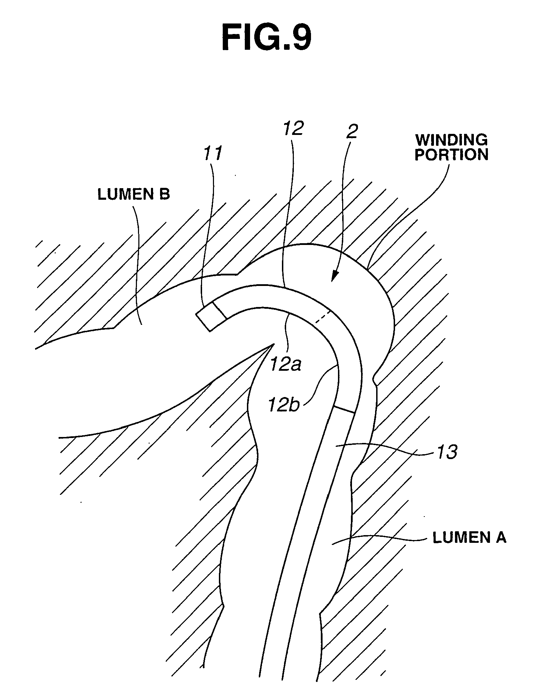

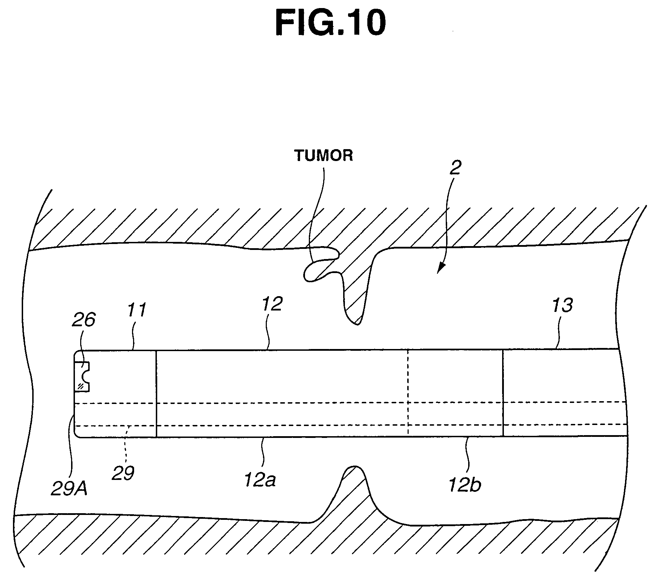

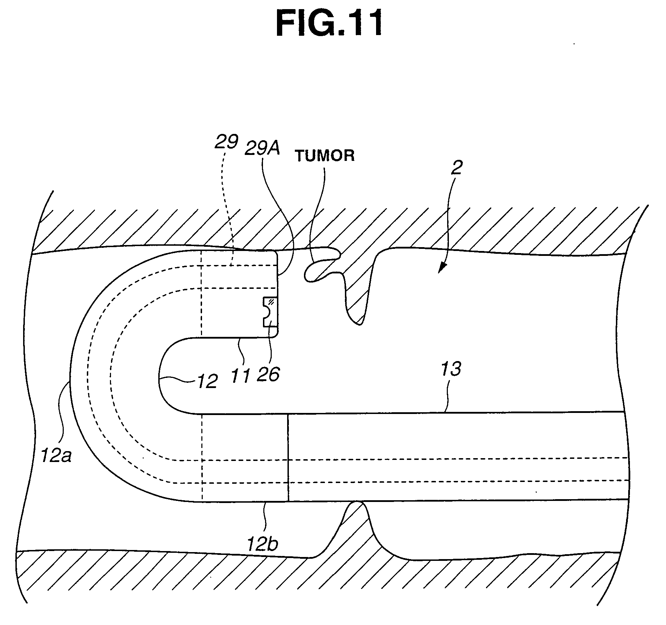

Figs. 1 to 11 relate to the embodiment of the invention. Fig. 1 is a construction diagram showing an endoscope according to an embodiment of the invention. Fig. 2 is a front view of a distal-end portion in Fig. 1. Fig. 3 is a schematic perspective view showing a construction of a bending portion in Fig. 1. Fig. 4 is a view of the bending portion in the direction indicated by the arrow B in Fig. 3. Fig. 5 is a view of the bending portion in the direction indicated by the arrow C in Fig. 3. Fig. 6 is a view of the endoscope in the direction indicated by the arrow A in Fig. 1. Fig. 7 is a schematic explanatory diagram in which a direction of an observation image displayed on a monitor is illustrated. Fig. 8 is a schematic explanatory diagram showing a state of an inserting portion having a bending portion bending at a small bend radius in a winding portion of a lumen. Fig. 9 is a schematic explanatory diagram showing a state of the inserting portion having the bending portion bending at a large bend radius in the winding portion of the lumen. Fig. 10 is a schematic diagram showing a state of the inserting portion having the distal-end portion near a target part within a lumen. Fig. 11 is a schematic diagram showing a state of the inserting portion having the bending portion bending from the state in Fig. 10 at a small bend radius.

As shown in Fig. 1, an endoscope 1 according to this embodiment of the present invention includes an elongated flexible inserting portion 2 and an operation section 3 on the proximal end side of the inserting portion 2. The endoscope 1 includes a flexible universal cord 4 extending from a side of the operation section 3. The universal cord 4 has, at the end, a connector section (not shown), which can be removably connected to a light source apparatus and video processor, not shown. The endoscope 1 is also connected to a suction apparatus, a front water-feeding apparatus and a fluid-feeding tank, not shown.

The inserting portion 2 includes a hard distal-end portion 11 at the distal end, a bendable bending portion 12 at the proximal end of the distal-end portion 11 and a long flexible tube 13 at the proximal end of the bending portion 12.

The operation section 3 includes a handle 3a on the inserting portion 2 side. The handle 3a is used to grasp the endoscope 1. An air/water feeding operation button 21 and a suction operation button 22 are provided above the handle 3a (on the proximal end side of the operation section 3). The air/water feeding operation button 21 is used for performing air-feeding operations and water-feeding operations. The suction operation button 22 is used for performing suction operations.

Multiple bending operation members 23 such as a knob and a lever are provided on the side of the handle 3a having the air/water feeding operation button 21 and the suction operation button 22. The bending operation members 23 are used for performing bending operations and can bend the bending portion 12 in multiple directions. According to this embodiment, as the multiple bending operation members 23, two bending operation members are provided which can bend the bending portion 12 in four directions including vertical and horizontal directions, as described later. The details will be described later.

Multiple remote switches 24 are provided at the top of the handle 3a. The remote switches 24 are used for remotely operating the video processor.

On the other hand, a therapeutic-instrument insertion opening 25 is provided below the handle 3a (on the proximal end side of the inserting portion 2). The therapeutic-instrument insertion opening 25 is an opening communicating with the therapeutic-instrument-through channel 29 (refer to Figs. 10 and 11). A forceps stopper, not shown, is removably attached to the end edge of the therapeutic-instrument insertion opening 25.

As shown in Fig. 2, an objective optical system 26, an air/water feeding nozzle 27, an illumination optical system 28, a distal-end opening 29A of the therapeutic-instrument-through channel 29 and a front water-feeding opening 30 are provided at the distal-end portion 11 of the inserting portion 2. The air/water feeding nozzle 27 is used for blowing a fluid such as water and air to a surface of the objective optical system 26 to clean the surface thereof. The front water-feeding opening 30 is used for cleaning a target part of a subject. An image pickup plane (image incident end face of an image transmitting optical system, such as an image guide and a relay lens, in an optical endoscope) of an image pickup unit is provided at an image-forming position of the objective optical system 26. An emission end face of a light guide, not shown, is disposed behind the illumination optical system 28. The dashed lines indicate bending operation wires, which will be described later, fixed at the distal-end portion 11.

Next, a detail construction of the bending portion 12 will be described.

As shown in Fig. 3, the bending portion 12 has multiple bend pieces (also called nodal rings) pivotably connected in series. In the bending portion 12, these multiple bend pieces are covered by bend blades cylindrically woven of thin wires, not shown. Furthermore, the bend blades are covered by bend rubber water-tightly thus forming the bending portion 12. Fig. 3 shows the bending portion 12 without the bend blades and the bend rubber.

The bending portion 12 comprises two parts including a distal-end side part 12a and a proximal-end side part 12b. In the distal-end side part 12a, a bend piece 31 is mounted at the distal-end portion 11. The bend piece 31 has a pair of pivoting portions 31a on the horizontal circumference of the back edge (edge on the proximal end side). In the description of this embodiment, the term, "horizontal" refers to a substantial lateral direction to Fig. 3 for simple description and one direction orthogonal to the axis of the bending portion 12. The term, "vertical" refers to a direction orthogonal to the horizontal direction and also orthogonal to the axis of the bending portion 12.

In the distal-end side part 12a, a distal end edge (called front edge, hereinafter) of a bend piece 32 is attached to the proximal end edge (called back edge, hereinafter) of the bend piece 31. The bend piece 32 has a pivoting portion 32a on the horizontal circumference of the front edge and has a pivoting portion 32b on the vertical circumference of the back edge. The pivoting portion 32a is connected to the pivoting portion 31a of the bend piece 31 through a pivotal axis 33. Thus, the bend piece 32 is vertically pivotable.

Similarly, in the distal-end side part 12a, a bend piece 34 has a pivoting portion 34a on the vertical circumference of the front edge and a pivoting portion 34b on the horizontal circumference of the back edge. The pivoting portion 34a is connected to the pivoting portion 32b of the bend piece 32 through a pivotal axis 35. Thus, the bend piece 34 is horizontally pivotably connected to the bend piece 32.

A bend piece 36 has pivoting portions 36a and 36b on the horizontal circumference of the front and back edges. The pivoting portion 36a is connected to the pivoting portion 34b of the bend piece 34 through a pivotal axis 37. Thus, the bend piece 36 is vertically pivotably connected to the bend piece 34.

Similarly, the bend piece 38 has a pair of pivoting portions 38a on the horizontal circumference of the front edge and a pair of pivoting portions 38b on the vertical circumference of the back edge. The pivoting portion 38a is connected to the pivoting portion 36b of the bend piece 36 through a pivotal axis 39. Thus, the bend piece 38 is vertically pivotably connected to the bend piece 36. Like the bend pieces 34, 36 and 38, a bend piece 40 is horizontally pivotably attached to the bend piece 38. A bend piece 41 is vertically pivotably attached to the bend piece 40. A bend piece 42 is vertically pivotably attached to the bend piece 41. Then, a bend piece 43 is horizontally pivotably attached to the bend piece 42. As described above, the bend pieces 40, 41, 42 and 43 are connected to the preceding bend pieces alternately vertically and horizontally pivotably.

As described above, the number of alternately pivotably connected bend pieces in the distal-end side part 12a of the bending portion 12 is not particularly limited. The bend piece 36 and the bend piece 41 may be eliminated, and the bend piece 34 and the bend piece 38 and the bend piece 40 and the bend piece 42 can be directly connected.

Under this construction, the distal-end side part 12a of the bending portion 12 can bend in four vertical and horizontal directions.

On the other hand, the proximal-end side part 12b of the bending portion 12 is different from the distal-end side part 12a. In the proximal-end side part 12b, bend pieces 44 to 49 are connected to the adjacent bend pieces through pivoting portions on the horizontal circumference of the front and back edges. Thus, the proximal-end side part 12b can only bend in two vertical directions. Apparently, the number of bend pieces is not particularly limited. A pair of pivoting portions on the horizontal circumference of the back edge and a pair of pivoting portions on the horizontal circumference of the front edge of the most distal-end bend piece 44 in the proximal-end side part 12b are connected. Thus, the bend piece 43 at the most proximal end of the distal-end side part 12a is connected to the bend piece 44.

In the distal-end side part 12a of the bending portion 12, a pair of bending operation wires (also called strings) 51 and 52 extend within the bending portion 12 and a flexible tube 13 along the vicinity of the horizontal pivotal axis through coil pipes (also called tightly-wound coils) 53 and 54. The pair of the bending operation wires 51 and 52 is pulled and loosened from the distal end side to undergo a bending operation. The coil pipes used in this embodiment have an uncompressible structure in which wires are tightly wound in a pipe form.

Distal-end portions of the bending operation wire 51 and 52 are fixed at two horizontally spaced points 51a and 52a of a proximal-end side frame body 11a of the distal-end portion 11. The proximal ends are connected to a bending operation mechanism (manually-controlled mechanism), not shown, within an operation body and are alternately pulled and loosened. The bending operation mechanism (manually-controlled mechanism) is connected to a bending operation member 23B, which will be described later.

The coil pipes 53 and 54 are fitted into the bending operation wires 51 and 52, respectively. The distal-end portions are fixed on horizontally opposed internal walls of the bend piece 44 in the proximal-end side part 12b. The proximal ends are fixed at a position fixing portion of the operation body. Thus, the coil pipes 53 and 54 accurately give, at the distal-end portion of the tightly wound coils, an amount of a pulling operation given at the pipe proximal ends to the bending operation wires 51 and 52.

Therefore, in the bending portion 12, the range of the distal-end side part 12a of the bending portion 12 is only bent horizontally to the left or right as shown in Fig. 4 in response to a selective operation on the bending operation wires 51 and 52.

Similarly, a pair of bending operation wires 55 and 56 extends within the bending portion 12 and the flexible tube 13 along the vicinity of the vertical pivotal axis through uncompressible coil pipes 57 and 58.

Distal-end portions of the bending operation wire 55 and 56 are fixed at two vertically spaced points 55a and 56a of the proximal-end side frame body 11a of the distal-end portion 11. The proximal ends are connected to a bending operation mechanism (manually-controlled mechanism), not shown, within the operation body and are alternately pulled and loosened. Another bending operation mechanism (manually-controlled mechanism) is connected to a bending operation member 23A, which will be described later.

The coil pipes 57 and 58 are fitted into the bending operation wires 55 and 56, respectively. The distal-end portions are fixed on vertically opposed internal walls of the flexible tube 13. The proximal ends are fixed at a position fixing portion of the operation body.

Therefore, in response to selective operations on the bending operation wires 55 and 56, pulling forces affect on both ranges of the distal-end side part 12a and proximal-end side part 12b of the bending portion 12 as shown in Fig. 5. Thus, the distal-end side part 12a and the proximal-end side part 12b can be vertically bent. As shown in Figs. 4 and 5, the bend radius of the bending portion 12, which is bent in accordance with an operation on the bending operation member 23A, is larger than the bend radius of the bending portion 12, which is bent in accordance with an operation on the bending operation member 23B.

As described above, when the bending operation wires 51 and 52 are selectively pulled, the distal-end side part 12a is only bent with the horizontally adjustable range. When the bending operation wires 55 and 56 are selectively pulled, the distal-end side part 12a and the proximal-end side part 12b of the bending portion 12 is bent entirely with the vertically adjustable range.

As shown in Figs. 4 and 5, in the axial direction of the inserting portion 2, a position on the distal end side of the bending portion 12 bent by the bending operation member 23A is substantially the same as the position on the distal end side of the bending portion 12 bent by the bending operation member 23B. Furthermore, in the axial direction of the inserting portion 2, a position on the back end side of the bending portion 12 bent in accordance with an operation on the bending operation member 23A is on the side closer to the hand side of the inserting portion 2 than the position on the back end side of the bending portion 12 bent in accordance with an operation on the bending operation member 23B.

When the bending operation wire 51 or 52 and the bending operation wire 55 or 56 are both pulled, the distal-end side part 12a and the proximal-end side part 12b are entirely and vertically bent. Furthermore, the distal-end side part 12a is only horizontally bent, and the bending portion 12 can be bent in a desired form thereby, which is extremely convenient for operations on the endoscope 1.

According to this embodiment, in at least one bending direction of the bending portion 12 to be operated by a bending operation member, which is closer to the handle 3a of the operation section 3, among multiple bending operation members 23, the bent part is longer than bent parts in other bending directions to be operated by other bending operation members. In other words, a bending range of the bending portion 12 bent in accordance with an operation of the bending operation member 23A is larger than the bending range of the bending portion 12 bent in accordance with an operation on the bending operation member 23B. In other words, the bent part of the bending portion 2 bent in accordance with an operation on the bending operation member 23A is longer than the bent part of the bending portion 12 bent in accordance with an operation on the bending operation member 23B.

In other words, more specifically, as shown in Fig. 6, in the endoscope 1, the bending operation member 23A is configured near the handle 3a of the operation section 3. Here, the bending operation member 23A is used for pulling and loosening the bending operation wire 55 or 56. Thus, in the endoscope 1, bending directions of the distal-end side part 12a and proximal-end side part 12b of the bending portion 12 can be operated by the bending operation member 23A at a position easily accessible by the thumb of the left hand grasping the handle 3a.

The bending directions of the distal-end side part 12a and proximal-end side part 12b of the bending portion 12 operated by the bending operation member 23A are directions in which the observation image 61 displayed on the monitor 60, as a display unit as shown in Fig. 7, is moved in the gravity direction (UP) or anti-gravity direction (DOWN) of the monitor 60.

Therefore, in the endoscope 1, when the inserting portion 2 is inserted into a body cavity, as described later, a bending operation, which is generally complicated, can be easily performed by the thumb of the left hand grasping the handle 3a.

On the other hand, in the endoscope 1, when a target part such as a tumor exists on a lumen wall, as described later, for example, the bending portion 12 must be bent at a small bend radius in order to capture the target part within the observation range. In this case, the bending operation is seldom and infrequently performed.

Therefore, in the endoscope 1, the bending operation member 23B far away from the handle 3a of the operation section 3 is defined as a bending operation member for pulling and loosening the bending operation wire 51 or 52 giving a pulling force only to the distal-end side part 12a of the bending portion 12. The bending operation member 23B can be also used by the right hand.

The bending direction of the distal-end side part 12a of the bending portion 12 bent by the bending operation member 23B is a direction in which the observation image 61 displayed on the monitor 60, as a display unit as shown in Fig. 7, is moved in the horizontal direction, that is, in the left direction (LEFT) or the right direction (RIGHT) of the monitor 60.

As described above, the bending operation member 23A is provided closer to the part, in the operation section 3, grasped by an operator than the bending operation member 23B.

According to this embodiment, as the multiple operation members 23, two of the bending operation member 23A close to and bending operation member 23B far from the handle 3a of the operation portion 3 are provided so that the bending portion 12 can be bent vertically and horizontally. However, a bending operation wires may be provided so as to bend the bending portion 12 diagonally and/or in other directions, and a bending operation member may be provided so as to perform bending operations in the directions.

The endoscope 1 having the above-described construction may be connected to a light source apparatus and a video processor and may also be connected to a suction apparatus, front water-feeding apparatus and a liquid-feeding tank for endoscopic examinations, for example. Then, an operator may grasp the handle 3a of the endoscope 1 as shown in Fig. 1 by the left hand and insert the inserting portion 2 into a body cavity of a subject, such as the colon, to observe a target part.

For example, the colon has many sharply wound portions between a lumen A and a lumen B as shown in Fig. 8. Here, for example, in the endoscope, when the flexible tube 13 is bent to make a loop in the lumen A, pushing the distal-end portion 11 only results in an increase in size of the loop. Thus, a force cannot be conducted to the distal-end portion 11, and the distal-end portion 11 does not advance. In this case, the operator can release the state by pulling the inserting portion 2 to straight and pushing the distal-end portion 11 again.

Here, when the inserting portion 2 is pulled, the distal-end portion 11 may be in the lumen B. In this case, the distal-end portion 11 is caught by the lumen B and does not come off. However, when the inserting portion 2 is pulled, the distal-end portion 11 may not be in the lumen B. In this case, the distal-end portion 11 comes off from the lumen A.

Therefore, an operator may try to insert the inserting portion 2 from the lumen A to the lumen B beyond the sharply wound portion. Here, when the operator performs a bending operation at a small bend radius on the bending portion 12 of the inserting portion 2 in the wound portion, only the range of the distal-end side part 12a is bent, and the bending portion 12 has a sharp angle.

Then, the distal-end portion 11 of the inserting portion 2 of the endoscope 1 pushes up the colon in the wound portion, and the inserting portion 2 cannot advance to a desired direction (called stick phenomenon). Thus, the operator needs a time for inserting the inserting portion 2 into the target part.

As described above, according to this embodiment, the endoscope 1 includes the bending operation member 23A closer to the handle 3a of the operation section 3 as a bending operation member for bending the bending portion 12 across both of the ranges of the distal-end side part 12a and the proximal-end side part 12b.

An operator can manipulate the bending operation member 23A by the thumb of the left hand grasping the handle 3a to bend the bending portion 12. Thus, in the endoscope 1, the bending operation wire 55 or 56 is pulled and loosened by a bending operation mechanism (manually controlled mechanism), and a pulling force is given to both ranges of the distal-end side part 12a and the proximal-end side part 12b. As a result, as shown in Fig. 9, the bending portion 12 can bend across both ranges.

Since, in the endoscope 1, the bending portion 12 is long, the inserting portion 2 does not push out and can easily pass through a wound portion, when the bending portion 12 is bent. As a result, the distal-end portion 11 of the inserting portion 2 can reach the next lumen B.

Thus, the endoscope 1 can have high operability since the inserting portion 2 in a body cavity can be easily bent, which is generally complicated, by the thumb of the left hand grasping the handle 3a.

Therefore, when the distal-end portion 11 is in the lumen B, an operator may pull the inserting portion 2, stretch it to the straight and push the distal-end portion 11 again. Thus, the distal-end portion 11 does not come off from the lumen A when the inserting portion 2 is pulled. As a result, the bending state of the inserting portion 2 can be released.

In this case, since the distal-end portion 11 is in the lumen B, the bending portion 12 may be moved so that the lumen B itself can be lifted from the reference center of the lumen B to adjust to a gentle angle. Thus, the inserting portion 2 of the endoscope 1 can easily pass therethrough.

As shown in Fig. 10, the distal-end portion 11 of the endoscope 1 can reach the target part.

Here, when the target part such as a tumor is on a lumen wall, an operator may bend the bending portion 12 at a smaller bend radius in order to capture the target part in the observation range.

Then, the operator may manipulate the bending operation member 23B to bend the bending portion 12 from the outside to the inside by extending the thumb of the left hand so as to bend. Thus, in the endoscope 1 as shown in Fig. 11, the bending operation wire 51 or 52 may be pulled or loosened by a bending operation mechanism (manually-controlled mechanism). As a result, a pulling force is given only to the distal-end side part 12a, and the bending portion 12 is bent.

Hence, the bending portion 12 of the endoscope 1 can be bent at a small bend radius in order to capture a target part within an observation range. As a result, when the inserting portion 2 of the endoscope 1 according to this embodiment is in a body cavity, a generally complicated bending operation can be easily performed by the thumb of the left hand grasping the handle 3a. Therefore, the inserted inserting portion 2 can be always highly operable, and a bending direction at a different bend radius can be advantageously selected as required.

According to this embodiment, the invention is applied to the endoscope 1, which is an electronic endoscope, containing an image pickup device in the distal-end portion 11 of the inserting portion 2. However, the invention may be applied to an electronic endoscope having a construction for picking up a subject image conducted by an image guide in the inserting portion 2 by using an image pickup device contained in the operation section 3. Alternatively, the invention may be applied to a so-called optical endoscope, which can observe a subject image conducted by an image guide by using an eyepiece above the operation section 3.

Furthermore, while one bending operation member is used to bend two directions including up and down directions or left and right directions in the description above, one bending operation member may be used to bend at least in one direction.

As described above, according to this embodiment, an endoscope can be provided which can maintain high operability while the endoscope is being inserted and can be selectively bent at a different bend radius as required.

It is noted that the present invention is not limited only to the embodiments as described hereinabove but various modifications or the like thereof can be made without departing from the scope of the present invention.

### Industrial Applicability

As described above, since high operability can be maintained while being inserted and a bending direction at a different bend radius can be selected as required, the invention can be also applied not only to medical endoscopes but also to industrial endoscopes.

## Claims

1. An endoscope, comprising:
an inserting portion to be inserted into a subject;
a bending portion provided in the inserting portion for being bent in accordance with a bending operation by an operator;
a first bending operation member provided in an operation section for bending the bending portion in at least one direction; and
a second bending operation member provided in the operation section for bending the bending portion in a different direction from the at least one direction,
wherein the first bending operation member is provided closer to a part grasped by the operator than the second bending operation member with respect to the operation section, and a bending range of the bending portion bent in accordance with an operation on the first bending operation member is larger than a bending range of the bending portion bent in accordance with an operation on the second bending operation member.

2. An endoscope according to Claim 1,
wherein a bending direction of the bending portion to be manipulated through the first bending operation member is the gravity direction or anti-gravity direction of display means displaying endoscopic observation images.

3. An endoscope according to Claim 1 or 2,
wherein, in an axial direction of the inserting portion, a position on a distal end side of the bending portion bent in accordance with an operation on the first bending operation member is substantially the same as the position on the distal end side of the bending portion bent by the second bending operation member.

4. An endoscope according to any one of Claims 1 to 3,
wherein, in an axial direction of the inserting portion, a position on a back end side of the bending portion bent in accordance with an operation on the first bending operation member is positioned closer to the hand side of the inserting portion than a position on the back end side of the bending portion bent in accordance with an operation on the second bending operation member.

5. An endoscope according to Claim 2,
wherein a bending direction of the bending portion to be manipulated through the second bending operation member is the horizontal direction of the display means displaying the endoscopic observation images.

6. An endoscope according to any one of Claims 1 to 5,
wherein the bending portion includes multiple bend pieces pivotably connected in series with each other, the multiple bend pieces are bent by the first bending operation member, and a part of the multiple bend pieces on the distal end side is bent by the second bending operation member.

7. An endoscope, comprising:
an inserting portion to be inserted into a subject;
a bending portion provided in the inserting portion for being bent in accordance with a bending operation by an operator;
a first bending operation member provided in an operation section for bending the bending portion in at least one direction; and
a second bending operation member provided in a position farther from a part grasped by the operator than the first bending operation member with respect to the operation section for bending the bending portion in a different direction from the at least one direction,
wherein a length of a bent part of the bending portion bent in accordance with an operation on the first bending operation member is longer than a length of a bent part of the bending portion bent in accordance with an operation on the second bending operation member.

8. An endoscope according to Claim 7,
wherein a bending direction of the bending portion to be manipulated through the first bending operation member is the gravity direction or anti-gravity direction of display means displaying endoscopic observation images.

9. An endoscope according to Claim 7 or 8,
wherein, in an axial direction of the inserting portion, a position on a distal end side of the bending portion bent in accordance with an operation on the first bending operation member is substantially the same as the position on the distal end side of the bending portion bent by the second bending operation member.

10. An endoscope according to any one of Claims 7 to 9,
wherein, in an axial direction of the inserting portion, a position on a back end side of the bending portion bent in accordance with an operation on the first bending operation member is positioned closer to the hand side of the inserting portion than a position on the back end side of the bending portion bent in accordance with an operation on the second bending operation member.

11. An endoscope according to Claim 8,
wherein a bending direction of the bending portion to be manipulated through the second bending operation member is the horizontal direction of the display means displaying the endoscopic observation images.

12. An endoscope according to any one of Claims 7 to 11,
wherein the bending portion includes multiple bend pieces pivotably connected in series with each other, the multiple bend pieces are bent by the first bending operation member, and a part of the multiple bend pieces on the distal end side is bent by the second bending operation member.

13. An endoscope, comprising:
an inserting portion to be inserted into a subject;
a bending portion provided in the inserting portion for being bent in accordance with a bending operation by an operator;
a first bending operation member provided in an operation section for bending the bending portion in at least one direction; and
a second bending operation member provided in a position farther from a part grasped by the operator than the first bending operation member with respect to the operation section for bending the bending portion in a different direction from the at least one direction,
wherein a bend radius of the bending portion bent in accordance with an operation on the first bending operation member is larger than a bend radius of the bending portion bent in accordance with an operation on the second bending operation member.

14. An endoscope according to Claim 13,
wherein a bending direction of the bending portion to be manipulated through the first bending operation member is the gravity direction or anti-gravity direction of display means displaying endoscopic observation images.

15. An endoscope according to Claim 13 or 14,
wherein, in an axial direction of the inserting portion, a position on a distal end side of the bending portion bent in accordance with an operation on the first bending operation member is substantially the same as the position on the distal end side of the bending portion bent by the second bending operation member.

16. An endoscope according to any one of Claims 13 to 15,
wherein, in an axial direction of the inserting portion, a position on a back end side of the bending portion bent in accordance with an operation on the first bending operation member is positioned closer to the hand side of the inserting portion than a position on the back end side of the bending portion bent in accordance with an operation on the second bending operation member.

17. An endoscope according to Claim 14,
wherein a bending direction of the bending portion to be manipulated through the second bending operation member is the horizontal direction of the display means displaying the endoscopic observation images.

18. An endoscope according to any one of Claims 13 to 17,
wherein the bending portion includes multiple bend pieces pivotably connected in series with each other, the multiple bend pieces are bent by the first bending operation member, and a part of the multiple bend pieces on the distal end side is bent by the second bending operation member.

19. An endoscope, comprising:
an inserting portion having a bending portion bendable in multiple directions; and
an operation section having multiple bending members for operating the bending portion of the inserting portion,
wherein a length of a bent part of the bending portion in at least one bending direction, which is operated by a operation member closer to a handle of the operation section among the multiple operation members, is longer than a length of a bent part in another bending direction, which is operated by another bending member.

20. An endoscope according to Claim 19,
wherein a bending direction of the bending portion operated by an operation member closer to the handle is a direction in which an observation image displayed on display means is moved in the gravity direction or anti-gravity direction of the display means.
